# EUROPEAN PATENT APPLICATION

(11) **EP 4 098 320 A1**
(43) Date of publication of application: **07.12.2022**
(21) Application number: 21748450.0
(22) Date of filing: 18.01.2021
(51) Int. Cl.: A61N 5/067, A61B 1/00, A61B 1/045

(54) **LIGHT/HEAT TREATMENT DEVICE HAVING THERMO-ENDOSCOPE**

(30) Priority: 31.01.2020 JP 2020014371
(71) Applicant: National University Corporation Tokai National Higher Education and Research System, Nagoya-shi, Aichi 464-8601 (JP)
(72) Inventor: UCHIDA, Hiroo, Nagoya-shi, Aichi 464-8601 (JP); MORIMOTO, Yuji, Tokorozawa-shi, Saitama 359-0042 (JP); NOMURA, Shinsuke, Tokorozawa-shi, Saitama 359-0042 (JP); OHYA, Jun, Tokyo 169-8555 (JP); OHARA, Mutsuki, Tokyo 169-8555 (JP); MASAMUNE, Ken, Tokyo 162-8666 (JP)
(74) Representative: Barth, Michael
(86) International application number: PCT/JP2021/001527
(87) International publication number: WO 2021/153319

(57) **Abstract**

A photothermal treatment device includes: irradiation means that irradiates a lesion tissue in a body cavity with heating light; a thermo-endoscope including endoscopic imaging means that images the inside of the body cavity and thermographic imaging means that images a surface temperature of a tissue in the body cavity as a thermal image; and control means (control personal computer (PC)) that controls an output and an irradiation time of the irradiation means based on temperature information of the thermal image obtained from the thermographic imaging means.

## Description

### TECHNICAL FIELD

The present invention relates to a photothermal treatment device having a thermo-endoscope.

### BACKGROUND ART

Conventionally, photothermal therapy (PTT) has attracted attention as a minimally invasive treatment method applicable to treatment of a malignant tumor that is difficult to be surgically extracted. This PTT is a treatment method in which a photosensitive pigment called a light absorbing agent is administered into a body to allow the agent to be accumulated in a target biological tissue, and then the light absorbing agent is excited by irradiation with laser light having a specific wavelength to cause cells of the target tissue to die. According to the photothermal treatment device, a lesion tissue can be effectively necrotized by being heated at a predetermined temperature and for a predetermined time corresponding to the type thereof, and the destruction of the surrounding healthy tissue can be prevented from being destroyed.

### CITATIONS LIST

### PATENT LITERATURE

Patent Literature 1: JP 2000-79089 A

### SUMMARY OF INVENTION

### TECHNICAL PROBLEMS

However, conventionally, the heating condition of the lesion tissue has been difficult to be accurately tracked and grasped, and thus the lesion tissue cannot be easily necrotized reliably. Meanwhile, a device is known in which thermographic imaging means is incorporated in an endoscope in order to measure a surface temperature of a tissue in a body cavity (Patent Literature 1: JP 2000-79089 A).

By using such thermographic imaging means, the heating condition of the lesion tissue can be tracked and grasped. However, it is very difficult for a doctor at an operating room to properly use the thermography. That is, because the doctor in the operating room needs to perform the photothermal treatment while viewing both an endoscopic image and a thermographic thermal image, it is extremely difficult to accurately grasp the correspondence relationship between the endoscopic image and the thermal image and to make a quick and accurate diagnosis. In addition, because the thermal image changes from moment to moment with the progress of light irradiation, there is complexity that output and time of the light irradiation have to be appropriately controlled correspondingly, which further increases the difficulty.

Therefore, an object of the present invention is to provide a photothermal treatment device that can easily grasp a temperature change of a lesion tissue and easily and appropriately heat the tissue at a predetermined temperature for a predetermined time.

### SOLUTION TO PROBLEMS

In order to solve the above problems, a photothermal treatment device according to the present invention includes: irradiation means that irradiates a lesion tissue in a body cavity with heating light; a thermo-endoscope including endoscopic imaging means that images an inside of the body cavity and thermographic imaging means that images a surface temperature of a tissue in the body cavity as a thermal image; and control means that controls an output and an irradiation time of the irradiation means based on temperature information of the thermal image obtained from the thermographic imaging means. ADVANTAGEOUS EFFECTS OF INVENTION

According to the present invention, the temperature change of a lesion tissue can be easily grasped, and the tissue can be easily and appropriately heated at a predetermined temperature for a predetermined time.

### BRIEF DESCRIPTION OF DRAWINGS

FIG. 1 is a side view of a photothermal treatment device according to an embodiment of the present invention.
FIG. 2 is a perspective view of the photothermal treatment device according to the embodiment of the present invention.

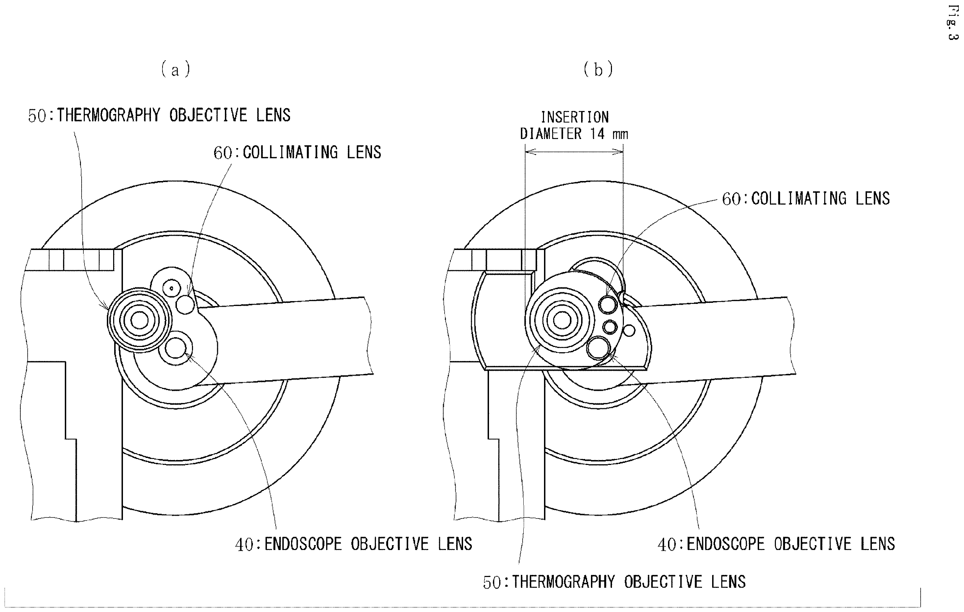

FIG. 3 is a front view of a probe tip of the photothermal treatment device.
FIG. 4A is a side view showing a state in which a thermo-endoscope probe is inserted into a trocar.
FIG. 4B is a conceptual view showing a state in which the trocar is inserted into an abdominal cavity of a rat and a lesion is irradiated with laser.
FIG. 5 is a view showing an image processing state of a thermal image.
FIG. 6 is a view showing a temperature control experiment of a rat liver tumor.
FIG. 7 is a view showing a depth of a tumor that can be treated when a target temperature during a photothermal treatment is changed.

### DESCRIPTION OF EMBODIMENT

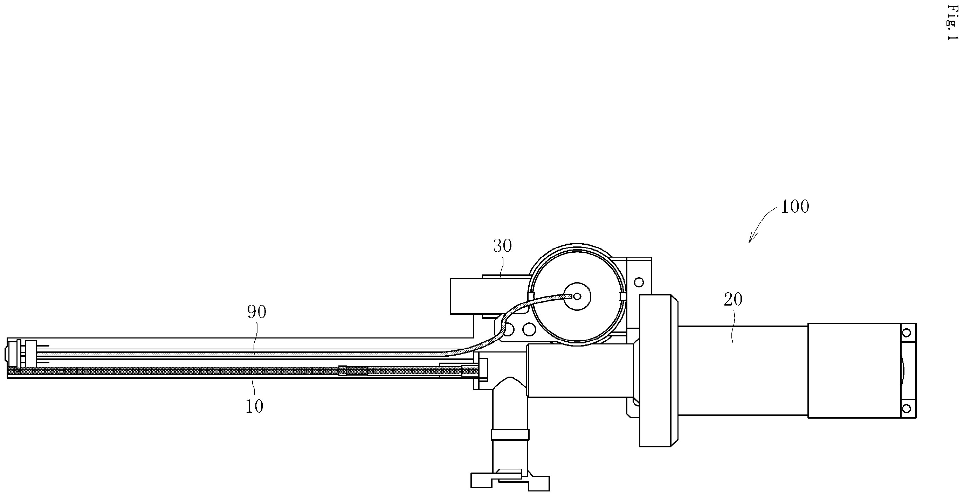

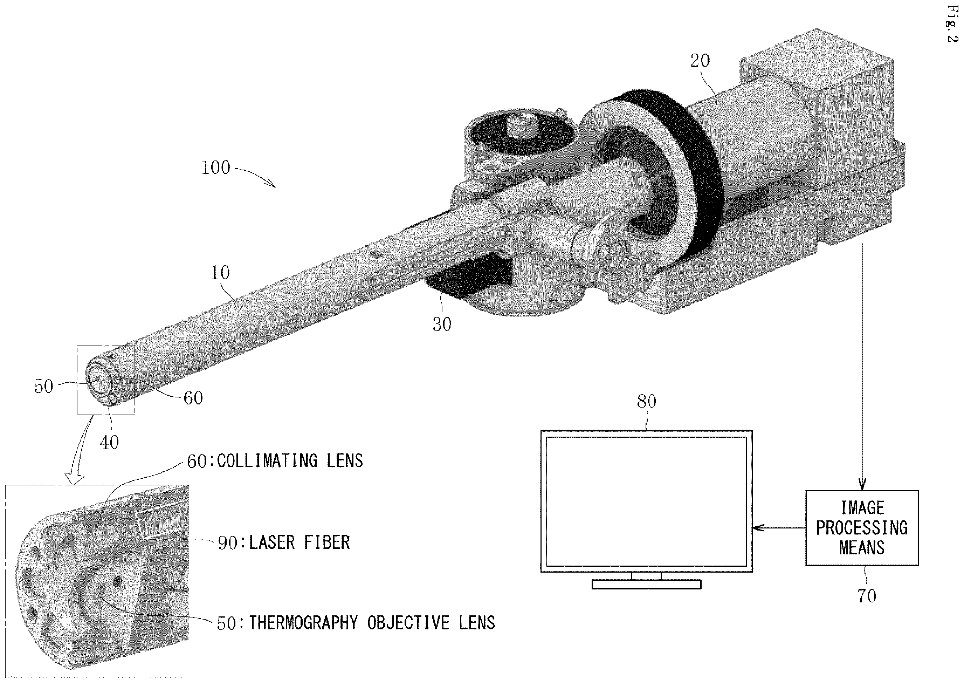

Hereinafter, a photothermal treatment device according to an embodiment of the present invention and a usage method thereof are described with reference to the drawings. As shown in FIGS. 1 and 2, a photothermal treatment device 100 according to the embodiment of the present invention includes an elongated probe 10, an operation part 20 disposed on a proximal end side of the probe 10, and a communication module 30 disposed between the probe 10 and the operation part 20.

The probe 10 has a cylindrical shape with a diameter of about 14 mm and a length of about 200 mm. However, the diameter and the length can be changed as necessary, and for example, the diameter of the probe 10 can be changed preferably in a range of 3 to 20 mm, and more preferably in a range of 5 to 16 mm. In the present embodiment, a diameter of about 14 mm is the most preferable size. The length of the probe 10 can also be changed preferably in a range of 150 to 250 mm, more preferably in a range of 180 to 220 mm. In the present embodiment, a length of about 200 mm is the most preferable size. At a distal end of the probe 10, an endoscope objective lens 40, an objective lens 50 for infrared thermography, and a collimating lens 60 for laser irradiation are disposed.

Infrared light emitted from an observation area in the body cavity is received by a thermopile element through the objective lens 50 for infrared thermography and an infrared filter behind the objective lens, and is converted into electric energy. Then, the electric energy is sent to image processing means 70 by the communication module 30.

A monitor 80 in FIG. 2 is configured to display an endoscopic image in the body cavity imaged by the endoscope objective lens and a charge-coupled device (CCD) element behind the endoscope objective lens 40 and a thermal image of the infrared thermography. The endoscopic image and the thermal image can be separately displayed on the monitor 80, but by switching a switch, the endoscopic image and the thermal image can be synthesized by the image processing means 70 and displayed on the monitor 80 in a superimposed state. In the synthesis, superimposition display is realized by performing adjustment by software so that the endoscopic image and the thermal image substantially overlap with each other in consideration of deviation of the optical axes of the endoscopic image and the thermal image.

A laser fiber 90 constituting heating light irradiation means together with the collimating lens 60 is connected to the rear side of the collimating lens 60. Then, laser light for photothermal treatment can be emitted to a lesion tissue through the laser fiber 90 and the collimating lens 60. The laser light for photothermal treatment herein includes laser light having a wavelength within a range from a visible light range to an infrared range.

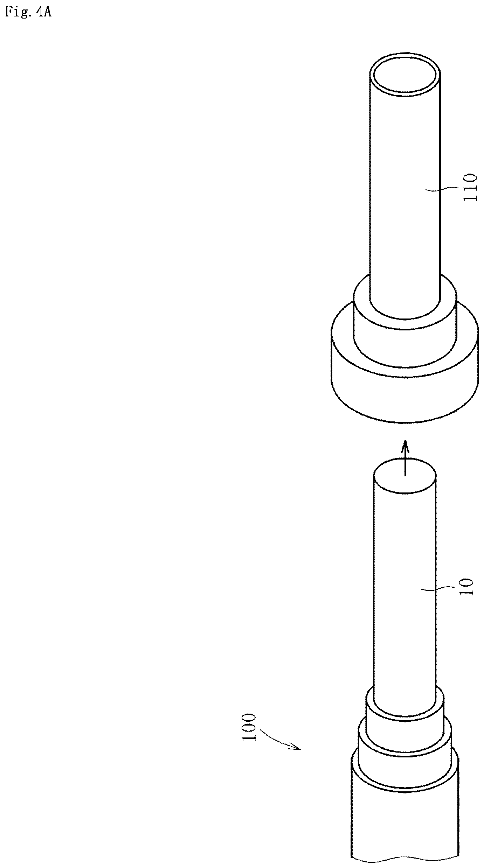

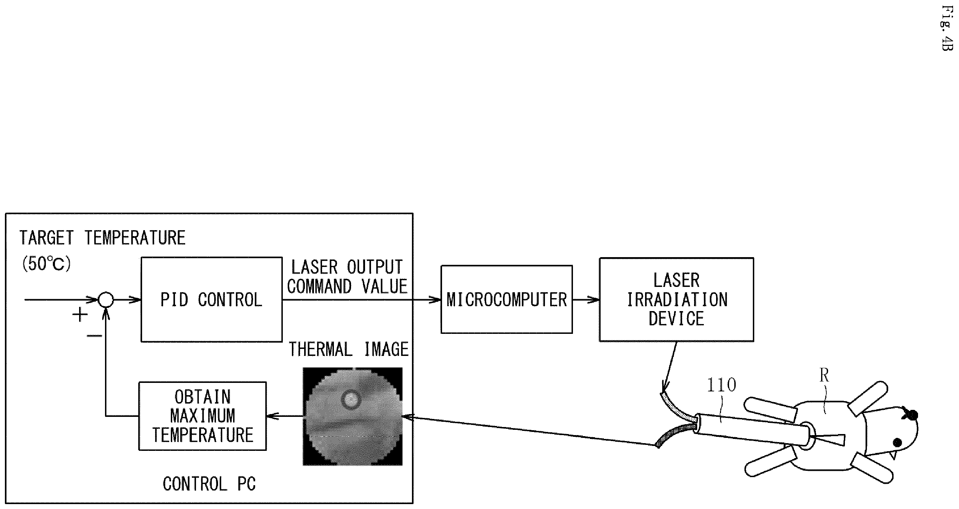

Next, a method of applying the photothermal treatment device 100 to a rat liver tumor is described. First, as shown in FIG. 4A, the probe 10 of the photothermal treatment device 100 is inserted into a trocar 110. Next, as shown in FIG. 4B, the trocar 110 is inserted into the abdominal cavity of the rat, and the target lesion tissue in which the light absorbing agent is accumulated is irradiated with the laser light. At the same time, the maximum temperature is acquired from the thermal image of the infrared thermography, and the laser output is subjected to Proportional-Integral-Differential (PID) control so that the target temperature of 50°C is achieved by the control personal computer (PC). The target temperature should be appropriately changed according to the type and the size of the target lesion tissue. In the present embodiment, the target temperature is set to 50°C, but the laser output can be PID-controlled preferably in a range of 43°C to 100°C, more preferably in a range of 43°C to 90°C, and most preferably at 80°C.

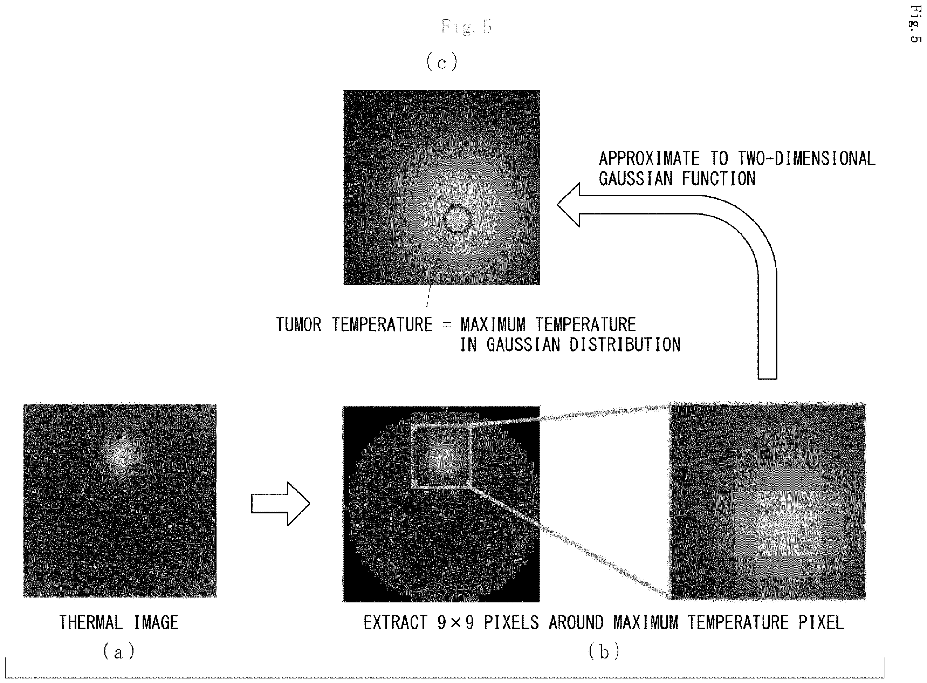

In the image processing means 70 in the control PC, as shown in FIG. 5 (b), 9 × 9 pixels around the maximum temperature pixel are extracted from the thermal image in FIG. 5 (a), and are approximated to a two-dimensional Gaussian function. Then, as shown in FIG. 5 (c), the maximum temperature of the Gaussian distribution is regarded as the tumor temperature, and the PID control is performed on the basis of the tumor temperature. Alternatively, the PID control may be performed based on the average temperature in a predetermined region (for example, in the 9 × 9 pixel region) of the thermal image.

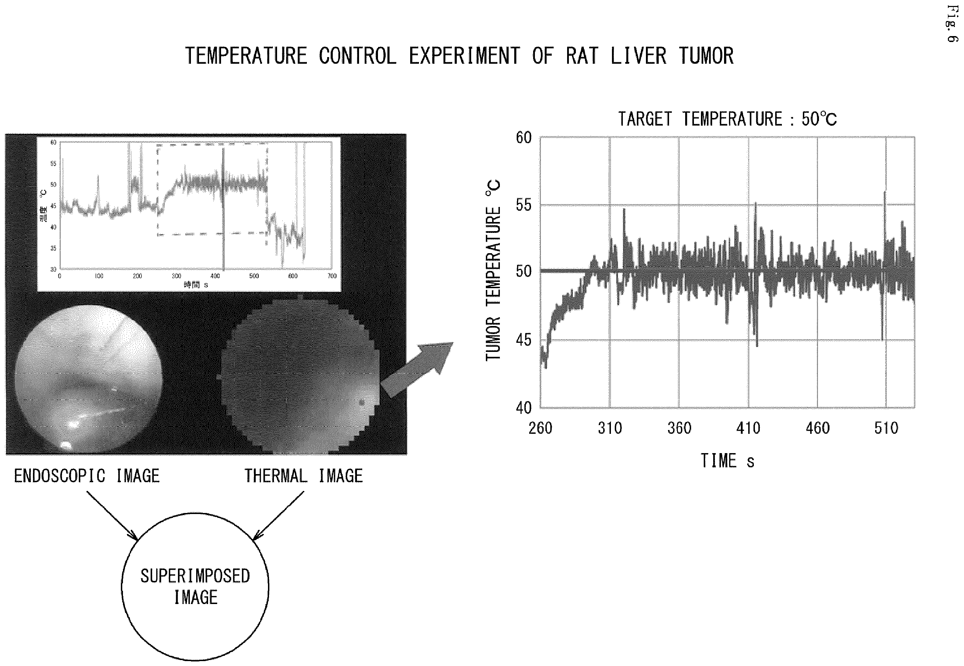

FIG. 6 shows a change in the tumor temperature when the rat liver tumor is actually subjected to photothermal treatment, and it has been confirmed that the tumor temperature can be controlled within a range of ±5°C of the target temperature of 50°C. Because the doctor in the operating room can easily confirm a state in which the thermal image is superimposed and displayed on the endoscopic image or a state in which both images are separately displayed on the monitor 80, the doctor can easily recognize a subtle temperature difference and can quickly and accurately position the laser light by accurate probe operation with respect to the target tissue in the body cavity in which the photosensitizer is excited.

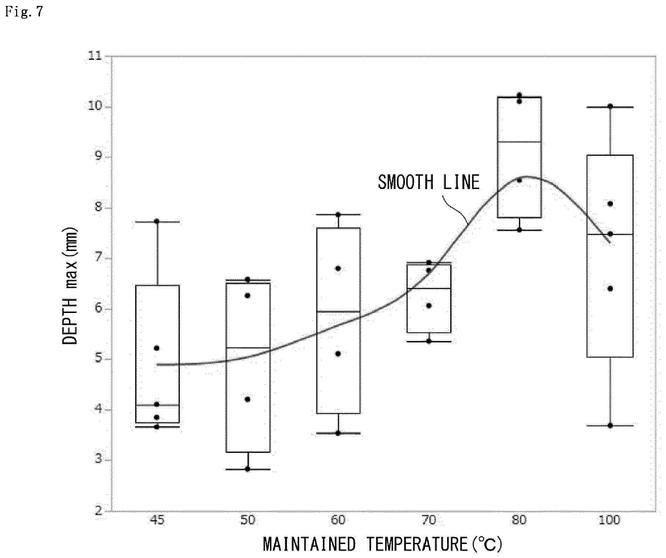

FIG. 7 shows the depth of tumor that can be treated (treatment depth) when the target temperature is changed (45°C to 100°C) at the time of actual photothermal treatment for the rat liver tumor. When the tumor temperature is changed from 45°C to 80°C, the treatment depth increases in proportion to the target temperature, the treatment depth becomes maximum (about 9 mm) at the target temperature of 80°C, and the treatment depth decreases at a higher target temperature. The doctor in the operating room can perform efficient tumor treatment while avoiding damage of normal tissue, by measuring the size of the tumor in advance by ultrasonic imaging or the like and changing the target temperature according to the depth of the tumor.

Even after the laser light is positioned, it has been conventionally necessary for the doctor to continue to appropriately control the output and the irradiation time of the laser corresponding to the thermal image that changes from moment to moment with the progress of laser irradiation. However, according to the photothermal treatment device of the present embodiment, the temperature change of the lesion tissue in the laser irradiation region can be easily grasped, and the output and the irradiation time of the laser can be automatically controlled by the control PC.

Therefore, the lesion tissue can be easily and appropriately heated at a predetermined temperature for a predetermined time. Thereby, the lesion tissue can be reliably necrotized by the photothermal treatment, and the destruction of the surrounding healthy tissue can be minimized.

Although the embodiment of the present invention has been described above, the present invention is not limited to the above embodiment, and various modifications can be made. For example, the thermographic imaging means may be equipped with a zoom lens to enlarge and display the thermal image of the lesion tissue. In addition, by coaxially arranging the optical axis of the endoscopic imaging means and the optical axis of the thermographic imaging means in the probe of the thermo-endoscope, the positional deviation of the image in the superimposed display of the endoscopic image and the thermal image can be minimized and the deviation of the display range in a case where both images are separately displayed can be minimized, and the image processing means can be simplified.

### REFERENCE SIGNS LIST

- 10: Probe
- 20: Operation part
- 30: Communication module
- 40: Endoscope objective lens
- 50: Thermography objective lens
- 60: Collimating lens
- 70: Image processing means
- 80: Monitor
- 90: Laser fiber
- 100: Photothermal treatment device
- 110: Trocar

## Claims

1. A photothermal treatment device comprising a thermo-endoscope, comprising:
irradiation means that irradiates a lesion tissue in a body cavity with heating light;
a thermo-endoscope including endoscopic imaging means that images an inside of the body cavity, and thermographic imaging means that images a surface temperature of a tissue in the body cavity as a thermal image; and
control means that controls an output and an irradiation time of the irradiation means based on temperature information of the thermal image obtained from the thermographic imaging means.

2. The photothermal treatment device according to claim 1, further comprising image processing means that displays an endoscopic image obtained from the endoscopic imaging means and the thermal image obtained from the thermographic imaging means in a superimposed manner on one display means.

3. The photothermal treatment device according to claim 1 or 2, wherein the control means controls the output and the irradiation time of the irradiation means based on an average temperature in a predetermined region of the thermal image.

4. The photothermal treatment device according to claim 1 or 2, wherein the control means approximately derives a two-dimensional Gaussian function from a temperature distribution in a predetermined region of the thermal image, and controls the laser output and the irradiation time of the irradiation means based on a maximum temperature of a Gaussian distribution obtained from the Gaussian function.

5. The photothermal treatment device according to claim 3 or 4, wherein the control means controls the output by Proportional-Integral-Differential (PID) control.

6. The photothermal treatment device according to any one of claims 1 to 5, wherein, in a probe of the thermo-endoscope, the endoscopic imaging means has an optical axis that is coaxially disposed with an optical axis of the thermographic imaging means.

7. The photothermal treatment device according to any one of claims 1 to 6, wherein the irradiation means emits, as heating light, laser light having a wavelength within a range from a visible light range to an infrared range.

8. The photothermal treatment device according to any one of claims 1 to 7, wherein the thermographic imaging means is infrared thermography.
